## Europäisches Patentamt
## European Patent Office
## Office européen des brevets

(11) Publication number: **0 098 073**
**B1**

(12)

# EUROPEAN PATENT SPECIFICATION

(45) Date of publication of patent specification: **14.12.88**

(51) Int. Cl.⁴: $A\ 61\ K\ 37/50,\ C\ 12\ N\ 9/96$

(21) Application number: **83303426.7**

(22) Date of filing: **14.06.83**

(54) Powdery pharmaceutical composition of myeloperoxidase.

(30) Priority: **25.06.82 JP 110532/82**

(43) Date of publication of application:
**11.01.84 Bulletin 84/02**

(45) Publication of the grant of the patent:
**14.12.88 Bulletin 88/50**

(84) Designated Contracting States:
**DE FR GB**

(56) References cited:
**FR-A-2 365 582**
**GB-A-2 066 260**
**GB-A-2 108 387**

(73) Proprietor: **THE GREEN CROSS CORPORATION**
**15-1, Imabashi-1-chome Higashi-ku Osaka-shi**
**Osaka 541 (JP)**

(72) Inventor: **Kobayashi, Takashi**
**5-5-508, Koshienguchi-5-chome**
**Nishinomiya-shi (JP)**
Inventor: **Yamana, Ryutaro**
**2-2-913, Kitamidorigaoka-1-chome**
**Toyonaka-shi (JP)**
Inventor: **Hasegawa, Eiichi**
**12-15, Hoshigaoka-2-chome**
**Hirakata-shi (JP)**

(74) Representative: **Harrison, David Christopher**
**et al**
**MEWBURN ELLIS & CO 2/3 Cursitor Street**
**London EC4A 1BQ (GB)**

Courier Press, Leamington Spa, England.

## Description

The present invention relates to a powdery pharmaceutical composition of myeloperoxidase comprising myeloperoxidase and an amount, enough to stabilize the myeloperoxidase, of citric acid or a salt thereof, as well as to process for producing said composition.

Myeloperoxidase (hereinafter, simply referred to as "MPO") is an enzyme which was first isolated in 1941 from human pus by Agner [Acta Physiol. Scand. 2, Suppl., 8 (1941)]. It is contained in a large quantity in myelogenous cells, particularly in polymorphonuclear leukocytes and monocytes, together with lysozyme, and its content amounts to 5% based on the weight of neutrophils. This enzyme is a basic hemo-protein having two iron atoms per one protein molecule and having a molecular weight of about 120,000—150,000 daltons. It belongs to the group of oxidoreductases. The physiological function of MPO is to kill or inactivate the pathogenic micro-organisms harmful to animals, such as bacteria, fungi and vira, in the presence of hydrogen peroxide and halide. The usefulness of MPO as a pharmaceutical is well known in that pharmaceutical compositions comprising MPO as a main ingredient exhibit a dramatic therapeutic effect when used for the treatment if infectious diseases with, for example, isonicotinic acid hydrazide (INH)-resistant mycobacterium tuberculosis.

Pharmaceutical compositions of MPO are usually offered as an injection or a topical composition having the form of a liquid or more preferably a freeze-dried powder, by sealing a unit dosage into an ampoule or a dividual container. However, pharmaceutical compositions comprising MPO as main ingredient are often unstable to freeze-drying, and they tend to decrease in MPO activity either at the time of freeze-drying or during the time after freeze-drying.

The present inventors conducted many studies with the aim of retaining the stability of pharmaceutical compositions comprising MPO as main ingredient during a long-term storage. As the result, it was found that the drop in MPO activity at the time of freeze-drying and the time-dependent decrease of MPO activity in the course of storage can be prevented and, at the same time, solubility of MPO compositions can be enhanced by adding citric acid or a salt thereof to MPO compositions.

It is the object of the present invention to provide a powdery MPO composition to which an amount, enough to stabilize the MPO, of citric acid or a salt thereof is added, as well as a process for producing said composition.

The citric acid or salt thereof used in the present invention is not critical, so far as it is physiologically acceptable. As the salt of citric acid, alkali metal salts such as the sodium salt and the potassium salt and alkaline earth metal salts such as the calcium salt and the magnesium salt can be referred to, for example. Of these salt, the sodium salt is particularly preferable.

The powdery MPO composition of the invention is preferably a freeze-dried product. The citric acid or salt thereof for stabilizing MPO is added before freeze-drying the aqueous solution of MPO, and it is not removed but is allowed to remain in the powder. Alternatively, it may also be added and mixed just after the freeze-drying. The amount of citric acid or salt thereof may vary depending on the form and concentration of MPO at the time of adding the citric acid or salt thereof. However, if it is added before the aqueous solution of MPO having a concentration of from 20 to 10,000 units/ml is freeze-dried, it should be added so that its concentration reaches a level of from 0.1 to 4.0 W/V%, preferably 0.3 to 2.0 W/V%. If it is added to a powdery material comprising from 200,000 to 500,000 units of MPO and a buffering salt, it should be added so that its concentration reaches a level of from 1.5 to 3,000 W/W%, preferably 4.5 to 1,500 W/W%. The proportion of citric acid or the salt thereof in the powdery MPO composition thus obtained is from 0.1 to 500 μg per one unit of MPO.

Next, the stabilizing effect on MPO will be explained with reference to Experimental Examples 1—4. Activity of MPO was determined by an improvement of the method of B. Chance et al. [Method in Enzymology, II, 764 (1955)] using guaiacol.

Experimental Example 1

Trisodium citrate, as a stabilizer, was added to 5 ml of an aqueous solution of MPO having a concentration of 100 units/ml before freeze-drying the aqueous solution, so that concentration of trisodium citrate reached 0.1, 0.5, 2.0 or 4.0 W/V%. After the MPO solution was freeze-dried, residual titer of the resulting mixture was measured (A) just after it had been freeze-dried and (B) after it had been stored at room temperature for 6 months. The results are summarized in Table 1.

# EP 0 098 073 B1

### TABLE 1
#### When the stabilizer was added before freeze-drying

| Stabilizer | Amount | | Residual titer | |
|---|---|---|---|---|
| | W/V% | μg/Unit of MPO | A | B |
| Trisodium citrate | 0.1 | 10 | 100 | 87 |
| | 0.5 | 50 | 100 | 100 |
| | 2.0 | 200 | 100 | 100 |
| | 4.0 | 400 | 100 | 100 |
| Control | 0 | 0 | 83 | 30 |

Experimental Example 2

Trisodium citrate, as a stabilizer, was added to 10 ml of an aqueous solution of MPO having a concentration of 10,000 units/ml before freeze-drying the solution, so that concentration of the citrate reached 0.1, 0.5, 2.0 or 4.0 W/V%. After the MPO solution had been freeze-dried, residual titer of the mixture was determined (A) just after it had been freeze-dried and (B) after it had been stored at room temperature for 6 months. The results are summarized in Table 2.

### TABLE 2
#### When the stabilizer was added before freeze-drying

| Stabilizer | Amount | | Residual titer (%) | |
|---|---|---|---|---|
| | W/V% | μg/Unit of MPO | A | B |
| Trisodium citrate | 0.1 | 0.1 | 100 | 93 |
| | 0.5 | 0.5 | 100 | 100 |
| | 2.0 | 2.0 | 100 | 100 |
| | 4.0 | 4.0 | 100 | 100 |
| Control | 0 | 0 | 100 | 75 |

Experimental Example 3

Five milliliters of an aqueous solution of MPO having a concentration of 100 units/ml was freeze-dried, just after which trisodium citrate was added as a stabilizer in the amount shown in Table 3. The mixture was stored at room temperature for 6 months, and then its residual titer (%) was determined. The results are shown in Table 3.

3

# EP 0 098 073 B1

### TABLE 3
### When the stabilizer was added just after freeze-drying

| Stabilizer | Amount | | Residual titer (%) |
|---|---|---|---|
| | W/W% | µg/Unit of MPO | |
| Trisodium citrate | 1.5 | 0.25 | 68 |
| | 4.5 | 0.75 | 87 |
| | 15.0 | 2.5 | 90 |
| | 45.0 | 7.5 | 94 |
| | 150.0 | 25.0 | 100 |
| | 450.0 | 75.0 | 100 |
| | 1,500 | 250.0 | 100 |
| | 3,000 | 500.0 | 100 |
| Control | 0 | 0 | 28 |

Experimental Example 4

One milliliter of an aqueous solution of MPO having a concentration of 10,000 units/ml was freeze-dried, just after which trisodium citrate was added and mixed as a stabilizer in the amount shown in Table 4. The mixture was stored at room temperature for 6 months, and then the residual titer (%) was measured. The results are shown in Table 4.

### TABLE 4
### When the stabilizer was added just after freeze-drying

| Stabilizer | Amount | | Residual titer (%) |
|---|---|---|---|
| | W/W% | µg/Unit of MPO | |
| Trisodium citrate | 1.5 | 0.15 | 95 |
| | 4.5 | 0.45 | 100 |
| | 15.0 | 1.5 | 100 |
| | 45.0 | 4.5 | 100 |
| | 150.0 | 15.0 | 100 |
| | 450.0 | 45.0 | 100 |
| | 1,500 | 150.0 | 100 |
| Control | 0 | 0 | 65 |

It is apparent from the results of these Experimental Examples that addition of citrate exerts a marked effect on the stability of MPO at the time of freeze-drying and on the stability of freeze-dried MPO composition in the lapse of time.

Next, the invention will be explained with reference to the following Examples in no limitative way.

Example 1

A dry powdery MPO composition was prepared by adding 0.5 W/V% of trisodium citrate to 5 ml of an aqueous solution of MPO having a concentration of 100 units/ml and then freeze-drying the mixture.

4

Example 2

A dry powdery MPO composition was prepared by adding 0.5 W/V% of trisodium citrate to 5 ml of an aqueous solution of MPO having a concentration of 10,000 units/ml and freeze-drying the mixture.

Example 3

A dry powdery MPO composition was prepared by freeze-drying an aqueous solution of MPO having a concentration of 100 units/ml and, just after it, adding 154 W/W% of trisodium citrate.

Example 4

A dry powdery MPO composition was prepared by freeze-drying an aqueous solution of MPO having a concentration of 10,000 units/ml and, just after it, adding 4.5 W/W% of trisodium citrate.

**Claims**

1. A powdery composition of myeloperoxidase comprising myeloperoxidase characterized in that it contains citric acid or a salt thereof as a stabilizer.

2. A powdery composition according to Claim 1, wherein the proportion of said citric acid or salt thereof is from 0.1 to 500 µg per one unit of myeloperoxidase.

3. A powdery composition according to Claim 1, wherein said salt of citric acid is the sodium salt, the potassium salt, the calcium salt or the magnesium salt.

4. A powdery composition according to Claim 3, wherein said salt of citric acid is the sodium salt.

5. A process for producing a powdery composition of myeloperoxidase which comprises adding an amount, enough to stabilize myeloperoxidase, of citric acid or a salt thereof to an aqueous solution of myeloperoxidase and freeze-drying the resulting mixture.

6. A process according to Claim 5 which comprises adding from 0.1 to 4.0 W/V% of citric acid or a salt thereof to an aqueous solution of myeloperoxidase having a concentration of from 20 to 10,000 units/ml and freeze-drying the resulting mixture.

7. A process according to Claim 5, wherein said salt of citric acid is the sodium salt, the potassium salt, the calcium salt or the magnesium salt.

8. A process according to Claim 7, wherein said salt of citric acid is the sodium salt.

**Patentansprüche**

1. Pulverförmige Myeloperoxidase-enthaltende Myeloperoxidase-Zusammensetzung, dadurch gekennzeichnet, daß sie Citronensäure oder ein Salz davon als Stabilisator enthält.

2. Pulverförmige Zusammensetzung nach Anspruch 1, in der das Verhältnis der Citronensäure oder des Salzes davon 0,1 bis 500 µg pro Myeloperoxidase-Einheit beträgt.

3. Pulverförmige Zusammensetzung nach Anspruch 1, in der das Salz der Citronensäure das Natrium-, Kalium-, Calciumsalz oder das Magnesiumsalz ist.

4. Pulverförmige Zusammensetzung nach Anspruch 3, in der das Salz der Citronensäure das Natriumsalz ist.

5. Verfahren zur Herstellung eines pulverförmigen Myeloperoxidase-Zusammensetzung, bei dem man eine zur Stabilisierung der Myeloperoxidase ausreichende Menge Citronensäure oder eines Salzes davon einer wäßrigen Myeloperoxidase-Lösung zusetzt und das entstehende Gemisch gefriertrocknet.

6. Verfahren nach Anspruch 5, bei dem man 0,1 bis 4,0 Gew./Vol.-% Citronensäure oder ein Salz davon einer wäßrigen Myeloperoxidase-Lösung mit einer Konzentration von 20 bis 10 000 Einheiten/ml zusetzt und das entstehende Gemisch gefriertrocknet.

7. Verfahren nach Anspruch 5, bei dem das Salz der Citronensäure das Natrium-, Kalium-, Calcium oder Magnesiumsalz ist.

8. Verfahren nach Anspruch 7, bei dem das Salz der Citronensäure das Natriumsalz ist.

**Revendications**

1. Composition en poudre de myéloperoxydase comprenant de la myéloperoxydase, caractérisée en ce qu'elle contient de l'acide citrique ou un sel de cet acide comme stabilisant.

2. Composition en poudre selon la revendication 1, dans laquelle la proportion d'acide citrique ou du sel de cet acide est de 0,1 à 500 µg par unité de myéloperoxydase.

3. Composition en poudre selon la revendication 1, dans laquelle ledit sel d'acide citrique est le sel de sodium, le sel de potassium, le sel de calcium ou le sel de magnésium.

4. Composition en poudre selon la revendication 3, dans laquelle ledit sel de l'acide citrique est le sel de sodium.

5. Procédé de préparation d'une composition en poudre de myéloperoxydase, qui comprend l'addition d'une quantité suffisante pour stabiliser la myéloperoxydase d'acide citrique ou d'un sel de cet acide à une solution aqueuse de myéloperoxydase et la lyophilisation du mélange ainsi obtenu.

6. Procédé selon la revendication 5, qui comprend l'addition de 0,1 à 4,0% en poids/volume d'acide citrique ou d'un sel de cet acide à une solution aqueuse de myéloperoxydase ayant une concentration de 20 à 10.000 unités/ml et la lyophilisation du mélange ainsi obtenu.

7. Procédé selon la revendication 5, dans lequel ledit sel d'acide citrique est le sel de sodium, le sel de potassium, le sel de calcium ou le sel de magnésium.

8. Procédé selon la revendication 7, dans lequel le sel d'acide citrique est le sel de sodium.